# EUROPEAN PATENT APPLICATION

(11) **EP 1 810 682 A1**
(43) Date of publication of application: **25.07.2007**
(21) Application number: 05779479.4
(22) Date of filing: 30.08.2005
(51) Int. Cl.: A61K 33/26, A61K 35/14, A61P 7/06

(54) **PREPARATION COMPRISING IONIC IRON AND HAEM IRON AND VARIANTS THEREOF FOR THE PROPHYLAXIS AND TREATMENT OF IRON DEFICIENCY**

(30) Priority: 30.08.2004 CU 18504
(71) Applicant: CENTRO NACIONAL DE BIOPREPARADOS, Bejucal, Provincia La Habana 6048 (CU); Laboratorios Medsol, Playa Ciudad de La Habana (CU)
(72) Inventor: GONZALEZ HERNANDEZ, RAUL R., Calle 238 No. 3318, Ciudad de la Habana, Habana 11 300 (CU); AZNAR GARCÍA, ELISA E., Calle 238 No. 3318 e/, Ciudad de La Habana, Habana 11 300 (CU); GONZALEZ PEREZ, MARITZA M., La Habana, La Habana (CU); HERNANDEZ GARCÍA, YENELA Y., La Habana, Cuba., La Habana (CU); HERRERA ÁVILA, YOSAIDA DE LA C., Ave. 35 No. 10818, Ciudad de La Habana, Habana (CU); GONZÁLEZ LAGO, MAYRA DE LA C., Saco No.216 Apt. 25, Ciudad de La Habana, Habana (CU)
(74) Representative: Gil-Vega, Victor
(86) International application number: PCT/CU2005/000006
(87) International publication number: WO 2006/024241

(57) **Abstract**

The present invention is related with the field of public health and particularly with a preparation that contains ionic and heme iron as powder to be used by oral route as tablets or in aqueous suspension for the prophylaxis and treatment of iron deficiency. The powder preparation contains between 6 to 26 % of ferrous fumarate and 74 to 94 % of hemoderivative in powder with a proportion between ionic iron and heme iron of 30 to 70 % and an amino acids contents of 20 to 30 µmol/100µmol of proteins. In 10 % aqueous suspension it contains a total iron concentration between 11 and 22 mg/mL. In tablets with mass of 500 to 700 mg it contains from 13 to 30 mg of total iron, from 0,2 to 0,25 mg of folic acid and from 45 to 55 mg of ascorbic acid. In addition to its effect on the prophylaxis and treatment off iron deficiency it can be used as restorative agent in convalescents and as therapeutic adjuvants.

## Description

### Field of the Invention

The present invention is related with the field of public health and particularly with a preparation that contains ionic and heme iron as powder to be used by oral route as tablets or in aqueous suspension for the prophylaxis and treatment of iron deficiency. The content of ionic iron derives from a ferrous salt and the heme iron from a hemoderivative in powder obtained from blood and other natural products.

### Description of the Related Art

Iron is a mineral indispensable for life although the daily requirements are low of around 1 mg per each 70 Kg of weight.

The biological functions of iron are known at least empirically since antiquity. Thus Jean-Paul CURTAY in his book NUTRITHERAPY: Scientific basis and medical practice, refers that the Greeks drank wine and the Romans water after keeping them in iron recipients with the objective of increasing the vigor.

The determination of iron contents in the human organism was reported for the first time by Widsswon E.M. et., al. (Cin. Sci., 1951, vol. 10,113). These papers indicated that the value of iron in tissues is approximately of 3.5 g of which 60-70 % is contained in the hemoglobin and the myoglobin and 20 % is stored in the liver, spleen and bone marrow in combinations that can be used if necessary. The rest that can constitute about 20 % is strongly fixed to tissues distributed in various enzymatic systems such as iron catalase, cytochromes, peroxidase, ferroproteins, transferrins, etc, (Boettcher E.W. et., al., Nature, 1958, Vol. 181.490).

In more recent papers (Marcel E. Conrad and Jay N. Umbreit in Blood Cell , Molecules and Diseases (2002),29(3): 336-355), reported that in the erythrocytes are concentrated 2500 mg of iron in the myoglobin and 300 mg in respiratory enzymes, 1000 mg as reserve and 4 mg circulating in plasma and they calculate the daily losses between 1 and 2 mg.

The human organism replaces its needs in part by endogenous iron derived from the destruction of aged red blood cells and in part by the iron supplied through food.

All iron metabolism regulations are established through the absorption of iron from food in the proximal portion of the small intestine, which has determined that most research on this oligoelement has been concentrated in clarifying all the mechanisms that intervene in the better use of this mineral contained in the food or that are supplied as supplement or medicament when required by its deficient status.

In spite of the wide knowledge available about the absorption and metabolism of iron and the low requirements of this mineral of which 1 mg daily should be absorbed by a normal adult person, it is not always achieved by the more physiological route that is its ingestion through food and this is demonstrated by the statistics of the International Institutions and Organizations that estimate in two thousand million the number of persons in the world that suffer iron deficiency constituting the most generalized lacking deficiency produced by one isolated element.

These realities determine the implementation of programs intended to protect higher risk populations groups that have greater requirements of this mineral such as children, pregnant women, and women in fertile age. These actions are fundamentally of prophylactic type, nevertheless, an important number of the persons develop an extreme grade of deficiency which is the iron deficiency anemia that constitutes 85 % of the causes of all anemias and that requires the application of therapeutic measures.

All the programs implemented to prevent iron deficiency are based on the use of iron salts obtained by synthetic route. However, these programs have had very low impact due to multiple causes among which are the following:
➢ Ionic iron contained in food of vegetable origin has to be reduced to bivalent iron by the gastric juice and then absorbed in the duodenum and first part of the jejunum thanks to the favorable effect of the pH in the first portion of the small intestine and stored specially in the liver.
➢ In vegetables, cereals and drinks of wide popular use such as tea, coffee and cacao there are substances such as phylates, polyphenols and metals that inhibit its absorption through the intestine.
➢ The measures to contra rest the effect of the inhibitors such as Vitamin C and the inclusion of meats in the diet is not at the reach of the most affected population..
➢ Protein deficiency and particularly essential amino acids decreases the bioavailability and affects the synthesis of the globulin chains which are protein components of the myoglobin.

When analyzing these factors evidences a predominant common factor which is the low bioavailability of ionic iron in the food or the one supplied through the synthetic salts to prevent and treat the deficiency of this mineral.

In investigations performed it has been observed up to 20 % of adverse reactions with the use of Ferrous fumarate {iron-protein anti anemic treatment (Trofin) for pediatric use. Elisa Aznar G., Raúl González H., Martha Moroña y Manuel González. Revista Mejicana de Ciencias Farmacéuticas. Vol. 29 No. 1 pp 18-21, 1998}.

The effects of the administration of ferrous or ferric iron salts is manifested in the gastrointestinal tract by the damages in the mucosa membrane like perforation and necrosis, allergic reactions like tachycardia and in some cases anaphylactic shock and cardiocirculatory collapse observed after the intramuscular administration (ferrodextran) and the intravenous route (ferrodextrin). ( Wollenberg, P. et al. Dependence of intestinal iron absorption on the valency state of iron. Arch Pharmacol., 336: 578-82, 1987).

To the inefficacy shown until now of the ionic iron in its different presentation forms are added the existing evidences about the role of iron in the formation of free radicals that participate in the oxidative stress. The high doses used in therapy and even for prophylaxis in some population groups such as pregnant women due to the low bioavailability rebounds negatively in two forms: the first is that a possible paracellular absorption route in the intestine is described that is not regulated by the enterocyte (Metabolismo del hierro. John L. Beard, Domingo J. Piñeiro. Deficiencia de Hierro: Desnutrición oculta en América Latina. Centro de estudios sobre Nutrición Infantil (CESNI). 13- 47) .1997) which can produce the entering of a ferrous iron to the blood plasma which is highly reactive. The second is that the iron in the colon participates in the reaction that originates free radicals that can influence in the mutations of the local cells and can be one of the factors that determines the appearance of colon cancer (Elizabeth K et al American Journal of Clinical Nutrition, Vol, 69, No, 2, 250-255. 1999).

Some modifications in its structure have been performed to solve these serious problems of the ferrous salts and one of them has been to modify the presentation of the ferrous salts to avoid the action of inhibitory factors before it reaches its specific absorption site. On example is constituted by the microencapsulated ferrous sulfate {New procedure for fortifying lactic products with microencapsulated ferrous sulfate. José R. Boccio, Marcela B. Zubillaga, Ricardo A. Caro, Carlos A. Gotelli, Mariano G. Gotelli, Ricardo Weill. Deficiencia de Hierro: Desnutrición oculta en América Latina. Centro de estudios sobre Nutrición Infantil (CESNI). 213-229 .1997 and Bioavailability and Stability of Microencapsulated Ferrous Sulfate in Fluid Milk: Studies in Mice. 1996. José R. Boccio, Marcela B. Zubillaga, Ricardo A. Cairo, CarlsoA. Gotelli, Mariano J. Goteli and Ricardo Weill. J. Nutr. Sci.Vitaminol., 42, 233-239}.

In spite of this innovative action the ferrous salt has not been devoid of its adverse effects and low efficacy { Treatment of anemia with microencapsulated ferrous fumarate plus ascorbic acid supplied as sprinkles to complementary (weaning) foods. 2001. Stanley Zlotkin, Paul Arthur, Kojo Yeboah Antwi and George Yeung. AJCN, Vol. 74, No. 6, 791-795} determined in the first place by the low bioavailability that obliges the use of high does that reinforce its astringency and irritability to the intestinal mucosa provoking abdominal pain, constipation, diarrheas, nauseas, vomiting and general disorders whose severity depends on the dose consumed and the time of the therapy. As alternative to the use of synthetic salts has been of scientific interest the use of iron of natural origin.

Even before having such wide knowledge about iron absorption and metabolism it was thought as a solution the use of iron of natural origin. In 1957 preparations based on ferritine were made ready. The most important reserve is ferroprotein, which entered rapidly in the clinical use due to its unquestionable advantages specifically related to its tolerance

Ferritine is extracted from bovine and equine spleen; it is hydrosoluble and thus can be administered by oral route, does not present undesirable effects in the digestive tract and also constitutes an iron reserve that is not activated by the intracellular proteins.

However its high cost and limited resources limit the manufacturing levels (Kadir, FH et. al. Haem binding to horse spleen ferritin and its effect on the rate of iron release. Biochem. J. 282: 867-70,1992).

Another source of natural iron that has been the objective of multiple investigations is heme iron (Ferroprotoporphyrin or prosthetic group of the hemoglobin)

The advantages of this iron were observed in the behavior of the bioavailability of iron in food when it contained both types of iron: ionic and heme in which case a better global bioavailability of dietetic iron was achieved.

These investigations have evidenced the presence of two types of receptors in the enterocytes one for heme iron which is the iron contained in the food of meat origin and another for the ionic iron that represents the nonheme iron. According to these results the route of the heme receptor is very efficient guaranteeing the internalization of iron in the enterocyte in two hours while for the nonheme or ionic are required at least 4 hours (Metabolismo del hierro. John L. Beard, Domingo J. Piñeiro. Deficiencia de Hierro: Desnutrición oculta en América Latina. Centro de estudios sobre Nutrición Infantil (CESNI). 13- 47).1997).

These results have served also for establishing strategies for the control of iron deficiency (Estrategia para la prevención y disminución de la prevalencia de la deficiencia de hierro a través de la alimentación. Miguel Layrisse y Maria Nieves Garcia-Casal.Deficiencia de Hierro: Desnutrición oculta en America Latina. Centro de estudios sobre Nutrición Infantil (CESNI). 163-175 .1997), which have demonstrated that the heme iron is absorbed until 25 % while the ionic iron barely surpasses 5 %.

These results have been used also as elements for establishing the bioavailability or iron consumed in a given country and defining the norms in each case according to the ingestion of one type iron or another (Current Methods of Estimating Dietary Iron Bioavailability, do not work in China. Shufa Du, Fengying Zhai, Youfa Wang And M. Popkin. 2000, J. Nutr. 130: 193-198).

In spite of these important contributions in the field of knowledge a preparation that could be used with therapeutic and prophylactic purposes was not offered and only a measure applicable through the diet. Although it contributed to demonstrate scientifically the advantages of the heme iron (The heme or protoporphyrin group that constitutes the prosthetic group of the hemoglobin, myoglobin and other enzymes of the P-450 enzymatic group) due to its rapid absorption, high bioavailability and not being submitted to the effect of inhibitory substances.

The most demonstrative antecedents for obtaining an heme iron based product were the studies of Smirnov et., al. (Revista Veterinaryia No. 4, 1969) whom demonstrated the feasibility of using bovine blood and gastric juice of horses in the elaboration of medicaments for animal use. Later González R. (Revista Salud Animal Vol. 1, No. 1, Año 1979) developed an hemolyzed product from the same components, but with substantial modifications in the technological process, to treat different diseases of domestic animals (Certificado de Autor de Invención con el No. 21 317, Título: Procedimiento de producción de Hemolizado con la Clasificación A61 K 35/14; 35/38 con fecha 20 de octubre de 1984).

These solutions only result of interest for the veterinary application since they do not fulfill with the requirements demanded for medicaments for human use.

The first practical experience about the use of heme iron in a program for prevention of iron deficiency in humans took place in Chile in the decade of the 80's where chocolate biscuits were fortified to be used in school children (Hemoglobin-Fortified Biscuits: Bioavailability and its effects on iron Nutriture in School Children. Manuel Olivares, Eva Hertrampf, Fernando Pizarro, Tomás Walter, Marisol Cayazzo, Sandra Llaguno, Patricia Chadud, Nelson Cartagena, Virginia Vega, Mirna Amar and Abraham Stekel..Arch, Latinoamer Ntr. 40: 209-220. 1990.

This result contributed a very positive proof but of limited scope since they did not propose an industrial solution for obtaining the heme iron by standardized technological methods and thus no standardized product was obtained.

The first product obtained in a liquid form based on heme iron was reported by González et., al. (Certificado de Autor de Invención con el No. 22 250, Title: FORMULA RECONSTITUYENTE PARA EL TRATAMIENTO DE LAS ANEMIAS, under classification A23J 1/06; A61K 35/12 dated 4 March 1994) obtained with a standardized technology with widely demonstrated therapeutic and prophylactic results:
➢ Estudio multicéntrico de un biopreparado con hierro ferroso de origen natural (Trofin) en ancianos con anemia.G.E. Aznar, H.R. González, B. Leyva, B. Muñoz, A. Daria. Geriatrika, 12 (10): 467-471. Pp 35-39 ,1996.
➢ Aplicación del Trofin en Geriatría. Elisa Aznar G., Raúl González H., Mayra Carrasco, Bárbara Muñoz y Bárbara Leyva.Revista Mejicana de Ciencias Farmacéuticas. Vol. 28 No. 1 pp 20-23, 1997.
➢ Tratamiento antianémico hierro-proteína (Trofin) para uso Pediátrico Elisa Aznar G., Raúl González H., Martha Moroña y Manuel González.Revista Mejicana de Ciencias Farmacéuticas. Vol. 29 No. 1 pp 18-21, 1998.
➢ Evaluación de la actividad teratogénica del Trofin, un antianémico de origen natural.Silvia Alvarez C. Ariadne Gutiérrez M. Carmen Valenzuela S. Y Elisa Aznar. Revista Mejicana de Ciencias Farmacéuticas. Vol. 30 No. 3 pp 19-23, 1999.
➢ Estudio clinico multicéntrico fase III del biopreparado proteico-mineral (Trofin) en niños con anemia ferropénica. Elisa Aznar, Raúl González, Martha Moroño, Manuel González, Olga García y Roberto Grau. Revista Mejicana de Ciencias Farmacéuticas. Vol. 34 No. 1 Enero-Marzo, 2003.

The high efficacy of this liquid preparation has additionally shown a high tolerance evidenced by a wide pharmacovigillance follow up {Sistema de farmacovigilancia y su aplicación en el seguimiento del producto hierro- proteina Trofin. Elisa Aznar, Aimé Pérez, Raúl González y Yuliet Pintueles. Revista Mejicana de Ciencias Farmacéuticas. Vol. 34, No. 2 Abril-Junio, 2003} which places it among the novel products in the fight against iron deficiency.

Other solutions have been reported with the use of heme iron (Obtención de una bebida instantánea a partir de hemoderivado para regímenes especiales de alimentación (Sarah Gutiérrez Rodríguez Universidad Politécnica de Valencia. Departamento de Tecnologías de Alimentos. Tesis Doctoral. Ciudad de la Habana. Cuba. 1998.) although the experiences of its use are very limited.

In spite of the important results with the liquid presentation, the growing need to expand the programs to segments needed of treatment and prophylaxis of anemia and with more attractive commercial presentations, it was necessary to obtain the solid forms which are easier to manipulate and of less manufacturing transportation cost.

Based on these criteria González et., al. developped a new product (Certificado de Autor de Invención under No. 22 599, Title: *HEMODERIVADO EN POLVOS PARA LA PROFILAXIS Y TRATAMIENTO DE LA DEFICIENCIA DE HIERRO* under classification A61K 35/14 ; A23J 1/06; A23L 1/08. Granted by resolution under number 1696/1999,dated 4 October 1999).

These products based on heme iron present the inconvenience of using only one of the intestinal receptors for iron absorption, which can contribute to their blocking by saturation while the other receptor for nonheme iron remains inactive which contributes to diminish bioavailability. Also in these products is not reported the presence of amino acids particularly the essential amino acids that could limit their biological effect by not synthesizing the proteins indispensable for hemoglobin synthesis and stimulate other organic functions that give other positive effects. Additionally in its composition do not appear other substances that increase absorption such as ascorbic acid or folic acid that result vital for fetal development particularly for the closure of the neural tube. Also the iron is not quantified by more reliable techniques.

### Detailed Description of the Invention

The objective of the present invention consists in developing a preparation containing heme iron and ionic iron that uses simultaneously both receptors of the enterocytes without risk of blocking by saturation which translates in superior results in the prophylaxis and treatment of iron deficiency without adverse reactions and obtaining other beneficial responses by the contents of amino acids, folic acid and ascorbic acid with a more reliable quantification of the iron contents.

Novelty of the invention consists in that a unique product was obtained that does not exist in the market, that has not been described before and that is distinguished by containing heme iron, ionic iron, amino acids, folic acid and ascorbic acid and presents the following characteristics:
- Contents of ionic iron represents at maximum 70 % of the total iron.
- Proportion between heme and ionic iron: 30-70 %
- Proportion of ferrous fumarate: 6-26 %
- Fine powder of brown-red color.
- Amino acids concentration: from 20 to 30 µmol/100 µL of proteins
- Dissolved at 10 % in water it has an iron concentration of 11 to 22 mg of iron per mL.
- In tablets of 500 to 700 mg it presents a concentration of 13 to 30 mg of iron with 0.2 to 0.25 mg of folic acid and 45 to 55 mg of ascorbic acid.

### THE FOLLOWING COMPONENTS ARE USED FOR ITS ELABORATION:

HEME IRON DEHYDRATE (ACCORDING TO PATENT No. 22 599)
- Fine powder of brown-red color.
- Contents of ferrous iron: 50-70 mg/gram of powder.
- Apparent density: 280 to 350 g / L.
- Humidity: 5 to 10 %.
- Total nitrogen: 8 to 13 mg %.
- Aminic nitrogen: 0.5 to 0,8 mg %.
- Dissolved at 10 % in water it forms a stable suspension with pH from 4 to 5.
- Slightly sweat and metallic taste.
- Drug-related odor.

**Table 1.- Other components present in the powders used as source of heme iron (According to patent No. 22 599)**

| **INDICATORS** | **Batch 6006** | **Batch 7001** | **Batch 7004** |
|---|---|---|---|
| ASHES (%) | 2.90±O,O1 | 3.06±0.012 | 2.41±0.02 |
| FAT (%) | 0,28±O.O2 | 0.23±0.01 | 0.24±0.01 |
| PROTEINS (%) | 47.52±0.21 | 49.98±0.18 | 49.40±0.13 |
| CALCIUM (mg/%) | 19.20±2.00 | 22.00±1.00 | 20.50±1.00 |
| MAGNESIUM (mg/%) | 7.50±0.60 | 6.84±1.40 | 6.20±1.50 |
| SODIUM (mg/%) | 850.00±50.00 | 983.00±29.00 | 791.50±40.00 |
| POTASIUM (mg/%) | 222.00±15.00 | 278.00±15.00 | 208.00±17.00 |
| PHOSPHORUS (mg/%) | 71.00±1.00 | 76.00±2.00 | 75.00±3.00 |
| CUPPER (mg/Kg) | 2.30±0.07 | 2.80±0.07 | 1.90±0.07 |
| ZINC (mg/Kg) | 20.90±0.40 | 18.90±0.30 | 22.40±0.10 |
| WATER ACTIVITY (Aw) | 0.256±0.001 | 0,238±0,002 | 0193±0.001 |

### FERROUS FUMARATE

➢ Aspect: Fine powder of brown-red color
➢ Molecular weight: 170 g/mol
➢ Chemical formula: C₄H₂FeO₄
➢ Contents of elemental iron: 33 %
➢ Solubility in water: Little soluble

### The technical advantages of the invention proposed consist in:

1.- The first and only preparation that has in its composition two types of iron: ionic iron derived from synthetic salts with low bioavailability and absorption causing adverse reactions that can affect up to 20 % of the patients and heme iron of natural origin of high bioavailability and absorption without producing adverse reactions.
2.- The presence of the two types of iron in the new preparation allows using both absorption routes present in the intestine through the enterocytes receptors specific for heme iron and for nonheme or ionic iron. Physiologically both receptors would function at the same time decreasing the risk of absorption blocking by saturation which occurs when using only one type of iron through a single receptor.
3.-In the new product the solubility of ferrous fumarate is increased which eliminates unpleasant effects of this product when administered alone in suspension which can provoke deposit in the teeth and the appearance of dark coloration.
4.-Adverse reactions such as constipation, diarrheas, nauseas or vomits that can occur in 20 % of the patients are eliminated. This favorable result is due to the increase of solubility, greater absorption by the simultaneous functioning of the enterocytes receptors for heme iron and ionic iron which conduces to the use of lower doses and additionally it has repercussion in less presence of free radicals in the intestine, which are associated with the appearance of colon cancer.
5.-Allows a better use by increasing the efficacy of one of the most important ferrous salts in the programs of prevention and treatment of iron deficiency by elevating the solubility and eliminating the adverse reactions.
6.-Presents good physical and microbiological stability due to the low contents of humidity; it has wide therapeutic safety margin, being an innocuous product with satisfactory organoleptic characteristics and high nutritional properties due to the biological value of its components.
7.-The contents of iron in 10 % suspension presents a total iron concentration of 11-22 mg / mL and of 13 to 30 mg in 500-700 mg tablets, which allows its use as supplement and medicament in all the population groups that may suffer from iron deficiency.
8.- The total contents of amino acids in the concentrations of 20 to 30 µmol/100 µL of proteins contributes not only to combat anemia but also to exert a favorable effect in the absorption of iron, it contributes to its better biological use since it allows the synthesis of proteins that form part of the hemoglobin structure and exerts a restorative effect in hypercatabolic status like in cancer patients in which a vital aspect is to improve the quality of life.
9.- The association of the ascorbic acid with the heme iron and ionic iron at a concentration of 45-55 mg/mL exerts a favorable action for increasing absorption contributing at the same time to maintaining the heme iron in ferrous form due to its reducing effect and the folic acid in the concentrations of 0,2-0,25 mg in the tablet of 500-700 mg has an additional effect for combating anemia due to other causes and constitutes a vital element in the prevention of anomalies during fetal development.

### Detailed description of the invention.

The dehydrate containing heme iron, ferrous fumarate salt, ascorbic acid and folic acid are mixed and homogenized in 5 Kg homogenizers in the proportions that guarantee concentrations of ionic iron of 30 to 70 % of the total iron and 30 to 70 % of heme iron. To achieve these iron concentrations were performed the calculations of the proportions in which should be mixed ferrous fumarate salt that contributes 33 % of the ionic iron and the hemoderivative powder as source of heme iron that contributes between 50 and 60 mg of iron per gram of powder. Based on this the final proportions are 6 to 26 % of ferrous salt and 74 to 94 % of the hemoderivative that contributes the heme iron.

The new product was dispensed in 235 mL flasks containing 15 grams for an iron concentration between 11 and 22 mg /mL.

For clinical trials in children the new product suspended in boiled water after tempering to room conditions was used.

In the trial were included 42 children of 6 to 9 years, of them 24 without anemia that received a prophylactic dose of 15 mg daily during 2 months and 18 with anemia that received a daily dose of 6 mg per Kg of life weight until clinical recovery or maximum two months of treatment. Hematological analysis performed were Hemoglobin by the cyanohemoglobin method and Hematocrit by capillary of microhematocrit before starting treatment and at one and two months respectively. Follow up of individual behavior was also performed to evaluate other clinical indicators and the appearance of adverse reaction. The following result was provided.

**Table 2.-GROUP WITHOUT ANEMIA TREATED PROPHYLACTICALLY**

| | HEMOGLOBIN g/L | | | HEMATOCRIT v/% | | |
|---|---|---|---|---|---|---|
| N | START | 1 MONTH | 2 MONTHS | START | 1 MONTH | 2 MONTHS |
| 24 | 116,5±5,37 | 118,0±11,0 | 117,0 ±8,0 | 38,0±2,49 | 38,0±2,24 | 38,0±2,24 |

During the 2 months only 3 children lowered hemoglobin value below 110 g/L which is the limit established as physiological for this age; in two the decrease was mild to 108 g/L and only one reached 95 g/L that can be considered as a moderate anemia.

If this result was important because a 96 % prophylaxis can be considered, more important even resulted that the levels of Hemoglobin and Hematocrit remained uniform during the two months of product consumption which can be interpreted as that the supply of both forms of iron in a single product follows the same absorption regulation mechanism then when they are administered individually. At present it is important to have adequate iron absorption but it is also important that it is not in excess, due to the collateral effects derived from the production of free radicals and its influence on oxidative stress.

No drug-related adverse reactions were observed either, although in prophylactic treatments is less frequent its appearance since lower doses are used. Nevertheless it is an important observation.

**Table 3.-GROUP WITH ANEMIA TREATED THERAPEUTICALLY**

| | HEMOGLOBIN g/L | | | HEMATOCRIT v/% | | |
|---|---|---|---|---|---|---|
| N | START | 1 MONTH | 2 MONTHS | START | 1MONTH | 2 MONTHS |
| 18 | 104,2±3,42 | 106,5±8,18 | 114,5±5,74 | 34,0±1,84 | 36,0 ±2,44 | 37,5±1,75 |

Of the treated group only 4 did not recover the normal hemoglobin values of 110 g/L, 3 reached up to 108 g/L and only one 95 g/L. In two of them occurred increases of 101 to 108 and 100 to 108 g/L respectively, one remained the same (108g/L) and only in one the hemoglobin decreased (101 to 95 g/L) although in the first month of treatment the child had reached 114 g/L.

The average hemoglobin increase in the group was 10 g/L, but one must consider that the anemia of these patients is mild (mean 104 g/L), which is closely related with absorbance, which is higher as lower is the hemoglobin contents. This result confirms the observation in the prophylaxis group that the absorption is regulated in function of the needs of the organism, which results of great interest for any product directed to combating iron deficiency.

Another extremely important result was the absence of adverse reactions, which contributed decisively to allowing completion of treatment and evidenced one of the more relevant advantages of this new product with respect to ionic salts about which are reported high incidences of diverse gastrointestinal disorders.

For the presentation in tablets were prepared mixtures with the following components:
➢ Hemoderivative in the form of powder as heme iron contribution in proportions of 74 to 94 %
➢ Ferrous fumarate as source of ionic iron in proportions of 6 to 26 %
➢ Ascorbic acid in amounts that guarantee concentration in the tablets of 45 to 55 mg.
➢ Folic acid in concentrations that guarantee in the tablets concentrations of 0.2 to 0,25 mg per tablet.

All the components considered as active materials were mixed adequately in mixers that guarantee adequate homogenization.

Two procedures were evaluated for manufacturing the tablets: direct compression and indirect compression.

Direct compression consists in directly compressing the powder mixture to conform the tablet without adding other components. This procedure did not result satisfactory thus it was discarded.

Indirect compression consists in adding other substances to the active principles that allow compressing the powder and obtaining the tablet.

This procedure allowed obtaining tablets of a mass between 500 and 700 mg with an iron concentration between 11 and 30 mg per tablet and fulfilling all the technological specifications.

With the objective of evaluating the new preparation in patients that require reconstituting and antianemic therapy was performed an investigation that included 60 patients with neoplasias, 30 with anemia and 30 without anemia under a rigorous selection based on inclusion and exclusion criteria as established by the Good Clinical Practices.

As fundamental recovery criteria was considered the integral evolution of a group of indicators for considering improvement of the quality of life of these treated patients in comparison with non treated controls.

Neoplastic patients with anemia were administered form 30 to 45 mL of suspension daily 2-3 times per day 15-20 minutes before foods during six months. The oncospecific treatment based on cytostatics or radiations continued as established. A systematic control was kept of the hematological parameters and the immunological profile.

Neoplastic patients without anemia were divided in two groups: one was administered the product prophylactically at a dose of 15-30 mL daily once or twice a day 15-20 minutes before foods during 6 months and another group remained as control. As main recovery criteria was considered the evolution of the hemoglobin and the continuity of the oncospecific treatment.

### The results obtained were the following:

Anemia patients:
➢ Hemoglobin increased from 94 g/L to 129 g/L at 90 days remaining without significant differences until 180 days after treatment
➢ 83.3% (25) responded with significant increases while only 16.7 % (3) did not respond, but they did not worsen their initial levels.
➢ Hemoglobin increase was of 35 g/L at 60 days allowing starting and fulfilling the oncospecific treatments in these patients.
➢ Increase of platelets and leukocytes count constituted an important results since these cells are indicators of defense mechanisms and it corresponded with the immunological results of the delayed hypersensitivity.
➢ 63.3% of the patients increased the serum proteins. This important blood biochemical indicator in 13.3 % (4) decreased and in 23.4% (7) of the patients it remained without variation.
➢ 86.6% of the patients increased the leukocytes and 100% increased the platelets.
➢ Satisfactory cellular immune response.
➢ The improvement in quality of life occurred in 83.3% of the patients. This constitutes the most important aspect for this type of patient since it integrates all the clinical indicators.

### Non anemic patients:

➢ It was demonstrated that 93.3% of the patients maintained and increased the hemoglobin values superior to 110 g/L.
➢ Hemoglobin remained in normal values during the oncospecific treatment.
➢ The patients of the Control Group decreased the hemoglobin after the application of the cytostatics with significant differences with the Group that received the product. These patients did not continue their treatment and it was necessary to suspend the oncospecific treatments they were receiving (cytostatic serums).

**Table 4.-Behavior of the treated patients.**

| **Total patients** | **End of oncospecific cycle** | **Interrupted treatment** | **Required transfusion** |
|---|---|---|---|
| 14 | 1 | 13 | 1 |

➢ Hematocrit and serum iron presented a behavior similar to the hemoglobin.

Iron determination was performed by the PHILIPS ANALYTICAL ATOMIC ABSORPTION method applying the technique of determining trace metals in food by atomic absorption spectroscopy. The preparation presents from 11-22 mg of iron per mL in 10 %aqueous suspension and from 11 to 33 mg per tablet of 500 to 00 mg of mass.

The determination of amino acids was performed in an LKB amino acids analyzer with total hydrolysis in the sample using 2 mL of the sample and applying 10 µL. The concentration was of 20 to 30 µmol/100 µL of proteins.

### PERFORMANCE EXAMPLES

### Example 1.

One of our experiments reflected is the following Table in which one of the heme iron based product in liquid presentation is compared evidencing the low results provided by ionic iron.

**Table 5.- Comparative study of the effect of ionic and heme iron**

| **CLINICAL PARAMETERS** | | **PATIENTS PERCENTAGE %** | |
|---|---|---|---|
| | | **GROUP I FUMARATE** | **GROUP II TROFIN^{®}** |
| Increase of: | | | |
| | Appetite | 42,8 % | 84,2 % |
| | Weight | 28,5 % | 76,5 % |
| | **Hemoglobin** | **58,0 %** | **95,2 %** |
| | **Serum Iron** | **72,0 %** | **100,0 %** |
| | Serum Proteins | 70,6 % | 90,1 % |
| **Adverse Reactions** | | **20 %** | **00,0** |
| Total Improvement | | 58 | 95,2 |

In spite of the poor results reported by these salts, they still play a preponderant role in the programs of fight against iron deficiency, which justifies the search for alternatives that contribute to increasing efficacy an aspect that constitutes the basis of the present invention.

### Example 2

The new product obtained basis its efficacy in the impact in combating the most generalized lacking disorder which is iron deficiency which requires presentation forms of easy use such as powder to restitute, tablets and granulates.

A wide study was performed of the solubility of the new product taking into consideration that the salt that is used in its composition is not soluble in water.

To evaluate the solubility of the different mixtures were used Osmometry and electric Conductivity techniques.

Osmometry: Over freezing temperature variation is proportional to the concentration of a substance and at the same time this measure is proportional to the amount of ions dissolved. When the samples show a concentration of particles that can not be read it is necessary to dilute them to be able to perform the reading.

Electric conductivity: It is proportional to the amount of ions dissolved.

In all the cases work was performed with supernatant. The samples dissolved from day zero, were homogenized manually on the day before the experiment to guarantee the integration to the supernatant of all the dissolve ions, while those that are dissolved in that moment were left 30 minutes at rest before starting the determinations.

**Table 6.- Conductivity results**

| **Standards** | **Conductivity** | | | | | | | |
|---|---|---|---|---|---|---|---|---|
| | **t=0** | **Average** | **t=7 days** | **Average** | **t=15days** | **Average** | **t=30days** | **Average** |
| 70 % Powder | 3.044 | 2.9685 | 2.676 | 2.678 | 2.82 | 2.8 | 3.291 | 3.3905 |
| | 2.893 | | 2.68 | | 2.78 | | 3.49 | |
| 50 % Powder | 2.089 | 2.1605 | 2.043 | 2.081 | 2.17 | 2.205 | 2.496 | 2.3785 |
| | 2.232 | | 2.119 | | 2.24 | | 2.261 | |
| 30% Powder | 1.675 | 1.627 | 1.546 | 1.5515 | 1.59 | 1.6 | 1.778 | 1.7715 |
| | 1.579 | | 1.557 | | 1.61 | | 1.765 | |
| 30% Fumarate | 0.497 | 0.5205 | 1.278 | 1.229 | 1.647 | 1.6175 | 1.837 | 1.8195 |
| | 0.544 | | 1.18 | | 1.588 | | 1.802 | |
| 50% Fumarate | 0.486 | 0.481 | 1.288 | 1.257 | 1.602 | 1.592 | 1.853 | 1.8715 |
| | 0.476 | | 1.226 | | 1.582 | | 1.89 | |
| 70% Fumarate | 0.485 | 0.493 | 1.302 | 1.31 | 1.669 | 1.6945 | 2.014 | 1.9655 |
| | 0.501 | | 1.318 | | 1.72 | | 1.917 | |
| 30% Sulfate | 3.58 | 3.61 | 3.4 | 3.42 | 4.03 | 3.94 | 4.23 | 4.23 |
| | 3.64 | | 3.44 | | 3.85 | | 4.23 | |
| 50% Sulfate | 5.39 | 5.215 | 5.1 | 5.025 | 5.93 | 5.955 | 6.09 | 6.11 |
| | 5.04 | | 4.95 | | 5.98 | | 6.13 | |
| 70% Sulfate | 5.98 | 5.98 | 6.53 | 6.545 | 6.87 | 7.06 | 7.32 | 7.365 |
| | 5.98 | | 6.56 | | 7.25 | | 7.41 | |

**Table 7.- Osmolarity results**

| | **Osmometry (mosmol/Kg)** | | | | | | | |
|---|---|---|---|---|---|---|---|---|
| Standards | **t=0** | **Average** | **t=7days** | **Average** | **t=15days** | **Average** | **t=30days** | **Average** |
| 70 % Powder | 236 | 235.5 | 220 | 215 | 260 | 258.5 | 278 | 287.5 |
| | 235 | | 210 | | 257 | | 297 | |
| 50 % Powder | 157 | 156 | 155 | 170 | 187 | 189 | 197 | 206.5 |
| | 155 | | 185 | | 191 | | 216 | |
| 30% Powder | 96 | 97.5 | 105 | 105 | 124 | 125 | 194 | 194 |
| | 99 | | 105 | | 126 | | 194 | |
| 30% Powder | 8 | 8 | 20 | 20 | 23 | 22 | 25 | 26 |
| | 8 | | 19 | | 21 | | 27 | |
| 50% Fumarate | 7 | 7 | 18 | 19 | 22 | 22 | 28 | 26.5 |
| | 7 | | 19 | | 22 | | 25 | |
| 70% Fumarate | 7 | 7 | 19 | 20 | 23 | 23 | 28 | 28 |
| | 7 | | 20 | | 23 | | 27 | |
| 30% Sulfate | 40 | 40 | 44 | 45 | 45 | 45.5 | 46 | 46 |
| | 40 | | 45 | | 46 | | 46 | |
| 50% Sulfate | 63 | 60.5 | 71 | 70 | 69 | 70.5 | 70 | 70 |
| | 58 | | 69 | | 72 | | 70 | |
| 70% Sulfate | 64 | 66 | 97 | 96 | 98 | 97 | 93 | 94 |
| | 68 | | 95 | | 96 | | 95 | |

**Table 8.- Composition of the mixtures and amount of powder/flask for each variant.**

| ***Powder** | **30 %** | **50%** | **70%** |
|---|---|---|---|
| **+Fumarate** | 59,8 g Powder | 42,72 g Powder | 25,6 g Powder |
| | 4,32 g Fumarate | 7,2 g Fumarate | 9,88 g Fumarate |
| **Quantity g/flask** | 4 g | 3,12g | 2,22 g |
| **Powder+ Sulfate** | 59,8 g Powder | 42,72 g Powder | 25,6 g Powder |
| | 4,7 g Sulfate | 7,8 g Sulfate | 10,96g Sulfate |
| **Quantity g/flask** | 4,03 g | 3,16 g | 2,29 g |

| | | | |
|---|---|---|---|
| *Powder containing heme iron | | | |

### Organoleptic characteristics of the preparation

Powder mixture plus ferrous fumarate at 30, 50 and 70% when resuspended with boiled drinking water took a intense brown red color, not observing lumps or residues of non dissolved product at simple sight in the moment in which they were resuspended. The reddish color became more intense during the 28 days the experiment lasted, while in the bottom of the flask deposited the non dissolved fumarate, which does not reintegrate totally to the liquid even when the flask is homogenized vigorously.

The mixture of Powders plus ferrous sulfate at 30, 50 and 70 % become a dark brown color when resuspended with drinking water. Non dissolved product lumps are observed on the surface of the liquid and in the walls of the flask not occupied by the product. These lumps can reach up to approximately 1 cm in diameter. During the 30 days of the experiment these lumps do not disappear and the color turns a lighter brown.

The following conclusions were reached from the results obtained of the electric conductivity, osmolarity and organoleptic characteristics:

### The following was concluded from these results:

➢ The powder samples plus sodium fumarate have a reddish color and a homogenous aspect while the samples of powders plus ferrous sulfate have a light brown color and do not dissolve completely.
➢ The salt of election as source of ionic iron in the new product is ferrous fumarate.
➢ As the concentration of fumarate increases in the samples decreases their solubility although at 30 days there is no significant difference between conductivity of the samples of powder plus fumarate at 30 and 50%.
➢ The ionic iron of the ferrous fumarate in the new product should represent between 30 and 50% of the total iron.
➢ In spite of the ferrous sulfate being more soluble in water than the ferrous fumarate it is not possible to use it as ionic iron source due to the changes of the characteristics of the new product obtained and because the use of osmometry is not feasible for evaluating the solubility behavior since with time its values decreases, thus at 30 days it was inferior to 15.

### Example 3

Tablets of 500-700 mg of mass were obtained with iron contents between 13 and 30 mg and with the technological parameters required for this type of presentation.

**Table 9.- Composition of the tablets of 500-700 mg**

| **COMPONENT** | **FUNCTION** | **QUANTITY (mg)** |
|---|---|---|
| Hemic iron powder | Active principle | 195-250 |
| Ferrous fumarate | Active principle | 39-90 |
| Vitamin C | Active principle | 45-55 |
| Folic acid | Active principle | 0.2-0,25 |
| Corn starch | Desintegrant | 115-170 |
| Carboxymethyl sodium starch | Desintegrant | 13-21 |
| Polyvinylpyrrolidine K-25 | Agglutinant | 13-21 |
| Polyethylene glycol- 6000 | Agglutinant | 3,5-5,2 |
| Magnesium stereate | Lubricant | 3,5-5,2 |
| Aerosyl 200v | Sliding agent | 2,6-3,85 |

### TECHNOLOGICAL RESULTS:

MASS = 500- 700 mg
HARDNESS= 7,0 kgf (Measured in Monsanto Equipment).
DESINTEGRATION ≤ 60 Minutes
IRON CONTENTS: 11-30 mg/ tablet

### Example 4

**Table 10.- Contents of amino acid in concentration of µmol/100 µL of proteins**

| **Amino acid** | **Contents** |
|---|---|
| Alanine (A) | 3,587±1.301 |
| Leucine (L) | 3.467±1.096 |
| Glutamic acid (E) | 2,726± 1.028 |
| Valine (V) | 2.724± 0.524 |
| Aspartic acid (D | 2.067±0.411 |
| Lysine (K) | 1.947± 0.616 |
| Glycine (G) | 1.892 ± 0.689 |
| Serine (S) | 1.596 ± 0.395 |
| Threonina (T) | 1.270 ± 0.372 |
| Phenylalanine (F) | 1.262 ± 0.387 |
| Arginine (T) | 1.157 ± 0.215 |
| Proline (P) | 1.108 ± 0.245 |
| Tyrosine (Y) | 0.587 ± 0.345 |
| Histidine (H) | 0.364 ± 0.199 |
| Isoleucine (I) | 0.151 ± 0.020 |
| Methionine (M) | 0.122 ± 0.084 |
| Cysteine (C) | Traces |
| Tryptophan (W) | Traces |

### Example 5

Improvement of Quality of Life in 83.3% of the cancer patients treated with the product constitutes a finding not reported in the literature for these pathologies characterized by a hypercatabolic status that compromises the different vital functions. This effect is also due to the specific antianemic effect determined by the contents of iron of high availability, to the contents of amino acids in total concentration of 20-30 µmol/100 µL of proteins which exert vital functions for the organism such as synthesis of hemoglobin, platelets and immunological activity.

**Table 11.- Quality of Life indicators in cancer patients.**

| LOCALIZATION | GROUP | HEMOGLOBIN g/L | PLATELETS Thousands /mL | LEUKOCYTES Thousands/mL |
|---|---|---|---|---|
| Breast | II A | ↑ 100% | ↑ 100 % | ↑ 100 % |
| | | . | | |
| | IIB | ↓ 100% | = 33% ↓ 67 % | = 67% ↓ 33 % |
| Ovary and Uterus | II A | ↑ 100% | ↑ 100 % | ↑ 100 % |
| | II B | ↓ 100% | ↓ 100 % | ↓ 67 % = 33% |
| Colon and Rectum | II A | ↑ 100% | ↑ 100 % | ↑ 100 % |
| | II B | ↓ 100 % | ↓ 100 % | =67 % ↓ 33 % |
| Bladder and Prostate | II A | ↑ 75 % = 25 % | ↑ 75 % = 25% | ↑ 75 % = 25% |
| | **II B** | **↓ 100 %** | **↓ 100 %** | **↓ 100 %** |

| | | | | |
|---|---|---|---|---|
| Group IIA: Patients that received the product prophylactically or therapeutically Group IIB: Control Group that did not receive the product | | | | |

Independently of the localization of the neoplasia in three of the principle indicators the response was positive in at least 75 % of the patients that received the product, while in the controls the worsening of said indicators was in most cases up to 100 %.

## Claims

1. Ionic and heme iron powder preparations for the prophylaxis and treatment of iron deficiency **characterized by** having in its composition from 6 to 26 % of ferrous fumarate, 74 to 96 % of hemoderivative in powder, a proportion of heme iron/ionic iron of 30 to 70 % and a contents of amino acids of 20 to 30 µmol/100 µL of proteins.

2. Ionic and heme iron powder preparation for the prophylaxis and treatment of iron deficiency that according to claim 1, **characterized** because said preparation in the 10 % suspension presentation contains a total iron concentration between 11 and 22 mg/mL.

3. Ionic and heme iron powder preparation for the prophylaxis and treatment of iron deficiency that according to claim 1, **characterized** because said preparation in the presentation of tablets of 500 a 700 mg contains a total iron concentration of 13 to 30 mg/ml of iron, from 0,2-0.25 mg of folic acid and from 45 to 55 mg of ascorbic acid.
